# EUROPEAN PATENT APPLICATION

(11) **EP 3 357 431 A1**
(43) Date of publication of application: **08.08.2018**
(21) Application number: 16850743.2
(22) Date of filing: 08.06.2016
(51) Int. Cl.: A61B 8/14

(54) **SOUND SPEED CALCULATION SYSTEM AND SOUND SPEED CALCULATION METHOD**

(30) Priority: 29.09.2015 JP 2015190725
(71) Applicant: FUJIFILM Corporation, Tokyo 106-8620 (JP)
(72) Inventor: YAMAMOTO, Hiroaki, Ashigarakami-gun Kanagawa 258-8538 (JP)
(74) Representative: Klunker IP Patentanwälte PartG mbB
(86) International application number: PCT/JP2016/067085
(87) International publication number: WO 2017/056566

(57) **Abstract**

Provided are a sound speed calculation system and a sound speed calculation method that can calculate a sound speed in a region of interest of a subject with high accuracy. A focus (F) of ultrasonic waves (w) is set in a portion that is farthest in the transmission direction of the ultrasonic waves (w) in a region of interest (35). Several second ultrasound transducers (51) to (69) receive ultrasound echoes from the focus (F). A sound speed in the region of interest (35) is calculated using ultrasound echo signals output from a first ultrasound transducer (60) that is closest to the focus (F), two second ultrasound transducers (51) and (69) that are other than the first ultrasound transducer, are located on straight lines (L2) passing through the focus (F) and the region of interest (35) of the subject, and have the first ultrasound transducer (60) interposed therebetween, and other ultrasound transducers (52) to (59) and (61) to (68) between the first ultrasound transducer (60) and the second ultrasound transducers (51) and (69).

## Description

### BACKGROUND OF THE INVENTION

### 1. Field of the Invention

The present invention relates to a sound speed calculation system and a sound speed calculation method.

### 2. Description of the Related Art

A technique which calculates a sound speed at two different points and calculates an average sound speed between the two points is considered in order to calculate the sound speed in a desired region of a subject (JP2010-207490A). In addition, a technique is considered which performs a process of directing an ultrasound probe to transmit ultrasonic waves such that a predetermined transmission focus is formed a plurality of times and calculates a sound speed on the basis of ultrasound echoes obtained from the transmission focus (JP2014-79568A).

In addition, the following techniques have been known: a technique that sets a transmission focus and a sound speed (JP2014-140410A); a technique that receives ultrasonic waves from two or more points in a subject as transmission focuses and determines an environmental sound speed (JP2013-208495A and JP2011-92686A); and a technique that determines a sound speed on the basis of first element data in a case in which a sound speed determination position is in the vicinity of the focus and determines the sound speed on the basis of second element data in a case in which the sound speed determination position is not in the vicinity of the focus (JP2014-68806A). Furthermore, there is a technique that changes a focal length depending on the depth of a region of interest (JP2003-93389A).

### SUMMARY OF THE INVENTION

However, JP2010-207490A to JP2003-93389A do not consider ultrasound echoes that pass through the region of interest of the subject and ultrasound echoes that do not pass through the region of interest of the subject. Therefore, in a case in which the sound speed in the region of interest of the subject is different from the sound speed in other regions of the subject, it is difficult to accurately calculate the sound speed in the region of interest of the subject.

An object of the invention is to calculate a sound speed in a region of interest of a subject with high accuracy.

A sound speed calculation system according to the invention includes: an acoustic probe in which a plurality of acoustic transducers are arranged; transmission driving means for driving the acoustic transducers such that the acoustic transducers transmit acoustic waves to a subject; acoustic image generation means for generating an acoustic image of the subject, using acoustic echo signals output from the acoustic transducers that transmit the acoustic waves to the subject and receive acoustic echoes from the subject; region-of-interest setting means for setting a region of interest in the acoustic image generated by the acoustic image generation means; focus setting means for setting a focus of the acoustic waves transmitted from several first acoustic transducers among the plurality of acoustic transducers in a portion that is farthest in a transmission direction of the acoustic waves in a region of interest of the subject which corresponds to the region of interest of the acoustic image; and sound speed calculation means for calculating a sound speed in the region of interest of the subject, using the acoustic echo signals output from a first acoustic transducer which is closest to the focus among several second acoustic transducers receiving the acoustic echoes from the focus set by the focus setting means, two second acoustic transducers which are other than the first acoustic transducer, are located on straight lines passing through the focus and the region of interest of the subject, and have the first acoustic transducer interposed therebetween, and other acoustic transducers between the first acoustic transducer and the second acoustic transducers.

The invention also provides a sound speed calculation method. That is, the method includes: allowing transmission driving means to drive a plurality of acoustic transducers included in an acoustic probe in which the acoustic transducers are arranged such that the acoustic transducers transmit acoustic waves to a subject; allowing acoustic image generation means to generate an acoustic image of the subject, using acoustic echo signals output from the acoustic transducers that transmit the acoustic waves to the subject and receive acoustic echoes from the subject; allowing region-of-interest setting means to set a region of interest in the acoustic image generated by the acoustic image generation means; allowing focus setting means to set a focus of the acoustic waves transmitted from several first acoustic transducers among the plurality of acoustic transducers in a portion that is farthest in a transmission direction of the acoustic waves in a region of interest of the subject which corresponds to the region of interest of the acoustic image; and allowing sound speed calculation means to calculate a sound speed in the region of interest of the subject, using the acoustic echo signals output from a first acoustic transducer which is closest to the focus among several second acoustic transducers receiving the acoustic echoes from the focus set by the focus setting means, two second acoustic transducers which are other than the first acoustic transducer, are located on straight lines passing through the focus and the region of interest of the subject, and have the first acoustic transducer interposed therebetween, and other acoustic transducers between the first acoustic transducer and the second acoustic transducers.

The region of interest of the subject has a shape that is surrounded by two radii and arcs of a circle and the portion that is farthest in the transmission direction of the acoustic waves is a center of the arc which is farthest from the acoustic probe in the region of interest of the subject.

In a case in which a straight line connecting the focus and the first acoustic transducer is a first straight line and a straight line connecting the focus and a first vertex that is farthest from the focus among four vertexes of the region of interest of the subject is a second straight line, for example, the sound speed calculation means sets the second acoustic transducer to an acoustic transducer located on the second straight line and calculates the sound speed in the region of interest of the subject, using the acoustic echo signals output from the first acoustic transducer, the second acoustic transducer, and other acoustic transducers.

In a case in which a straight line connecting the focus and a third acoustic transducer that is farthest from the focus among the plurality of acoustic transducers is a third straight line, for example, the sound speed calculation means sets the second acoustic transducer to an acoustic transducer located on the second straight line and calculates the sound speed in the region of interest of the subject, using the acoustic echo signals output from the first acoustic transducer, the second acoustic transducer, and other acoustic transducers, in a case in which a first angle formed between the first straight line and the second straight line is less than a second angle formed between the first straight line and the third straight line. The sound speed calculation means sets the second acoustic transducer to the third acoustic transducer and calculates the sound speed in the region of interest of the subject, using the acoustic echo signals output from the first acoustic transducer, the second acoustic transducer, and other acoustic transducers, in a case in which the first angle is greater than the second angle.

The several second acoustic transducers are a maximum number of acoustic transducers that are capable of receiving the acoustic echoes used to calculate the sound speed in the region of interest of the subject from the set focus at the same time. In a case in which a straight line connecting the focus and a fourth acoustic transducer that is farthest from the focus among the several second acoustic transducers is a fourth straight line, for example, the sound speed calculation means sets the second acoustic transducer to the acoustic transducer located on the second straight line and calculates the sound speed in the region of interest of the subject, using the acoustic echo signals output from the first acoustic transducer, the second acoustic transducer, and other acoustic transducers, in a case in which a first angle formed between the first straight line and the second straight line is less than a third angle formed between the first straight line and the fourth straight line. The sound speed calculation means sets the second acoustic transducer to the fourth acoustic transducer and calculates the sound speed in the region of interest of the subject, using the acoustic echo signals output from the first acoustic transducer, the second acoustic transducer, and other acoustic transducers, in a case in which the first angle is greater than the third angle.

The several second acoustic transducers include a fifth acoustic transducer. The fifth acoustic transducer is configured such that a difference between a first distance between the focus and the first acoustic transducer and a second distance between the focus and the fifth acoustic transducer is closest to a predetermined detection limit width. In a case in which a straight line connecting the focus and a fourth acoustic transducer that is farthest from the focus among the several second acoustic transducers is a fifth straight line, for example, the sound speed calculation means sets the fifth acoustic transducer to the acoustic transducer located on the second straight line and calculates the sound speed in the region of interest of the subject, using the acoustic echo signals output from the first acoustic transducer, the second acoustic transducer, and other acoustic transducers, in a case in which a first angle formed between the first straight line and the second straight line is greater than a fourth angle formed between the first straight line and the fifth straight line. The sound speed calculation means sets the second acoustic transducer to the fifth acoustic transducer and calculates the sound speed in the region of interest of the subject, using the acoustic echo signals output from the first acoustic transducer, the second acoustic transducer, and other acoustic transducers, in a case in which the first angle is greater than the fourth angle.

The several second acoustic transducers are a maximum number of acoustic transducers that are capable of receiving the acoustic echoes used to calculate the sound speed in the region of interest of the subject from the set focus at the same time. In a case in which a straight line connecting the focus and a third acoustic transducer that is farthest from the focus among the plurality of acoustic transducers is a third straight line, a straight line connecting the focus and a fourth acoustic transducer that is farthest from the focus among the several second acoustic transducers is a fourth straight line, and a first angle formed between the first straight line and the second straight line is greater than a third angle formed between the first straight line and the fourth straight line, for example, the sound speed calculation means sets the second acoustic transducer to the fourth acoustic transducer and calculates the sound speed in the region of interest of the subject, using the acoustic echo signals output from the first acoustic transducer, the second acoustic transducer, and other acoustic transducers, in a case in which the first angle formed between the first straight line and the second straight line is less than a second angle formed between the first straight line and the third straight line. The sound speed calculation means sets the second acoustic transducer to the third acoustic transducer and calculates the sound speed in the region of interest of the subject, using the acoustic echo signals output from the first acoustic transducer, the second acoustic transducer, and other acoustic transducers, in a case in which the first angle is greater than the second angle.

The several second acoustic transducers are a maximum number of acoustic transducers that are capable of receiving the acoustic echoes used to calculate the sound speed in the region of interest of the subject from the set focus at the same time. The several second acoustic transducers include a fifth acoustic transducer. The fifth acoustic transducer is configured such that a difference between a first distance between the focus and the first acoustic transducer and a second distance between the focus and the fifth acoustic transducer is closest to a predetermined detection limit width. In a case in which a straight line connecting the focus and a third acoustic transducer that is farthest from the focus among the plurality of acoustic transducers is a third straight line, a straight line connecting the focus and a fourth acoustic transducer that is farthest from the focus among the several second acoustic transducers is a fourth straight line, a straight line connecting the focus and the fourth acoustic transducer that is farthest from the focus among the several second acoustic transducers is a fifth straight line, and a first angle formed between the first straight line and the second straight line is less than a third angle formed between the first straight line and the fourth straight line, for example, the sound speed calculation means sets the second acoustic transducer to the acoustic transducer located on the second straight line and calculates the sound speed in the region of interest of the subject, using the acoustic echo signals output from the first acoustic transducer, the second acoustic transducer, and other acoustic transducers, in a case in which the first angle formed between the first straight line and the second straight line is greater than a fourth angle formed between the first straight line and the fifth straight line. The sound speed calculation means sets the second acoustic transducer to the fifth acoustic transducer and calculates the sound speed in the region of interest of the subject, using the acoustic echo signals output from the first acoustic transducer, the second acoustic transducer, and other acoustic transducers, in a case in which the first angle is less than the fourth angle.

For example, the acoustic probe is a convex acoustic probe in which the plurality of acoustic transducers are arranged in an arc shape.

The acoustic probe may be a linear acoustic probe. In this case, the region of interest of the subject has a rectangular shape and the portion that is farthest in the transmission direction of the acoustic waves is a center of a side that is far away from the acoustic probe in the transmission direction of the acoustic waves in the region of interest of the subject.

According to the invention, an acoustic image of the subject is generated and the region of interest is set in the generated acoustic image. The focus of the acoustic waves is set in the portion that is farthest in the transmission direction of the acoustic waves in the region of interest of the subject which corresponds to the region of interest of the acoustic image. In a case in which the acoustic probe transmits the acoustic waves, acoustic echoes are generated from the focus. In a case in which the ultrasound transducers receive the acoustic echoes, the ultrasound transducers that have received the ultrasound echoes output ultrasound echo signals. The sound speed in the region of interest of the subject is calculated using the ultrasound echo signals output from the first acoustic transducer that is closest to the focus, the second acoustic transducers that are located on the straight lines passing through the focus and the region of interest of the subject and have the first acoustic transducer interposed therebetween, and other acoustic transducers between the first acoustic transducer and the second acoustic transducers. The sound speed in the region of interest of the subject is calculated on the basis of the ultrasound echoes that pass through the region of interest of the subject, without using the ultrasound echoes that do not pass through the region of interest of the subject. Therefore, it is possible to calculate the sound speed with high accuracy.

### BRIEF DESCRIPTION OF THE DRAWINGS

Fig. 1 is a block diagram illustrating the electric configuration of an ultrasound diagnostic apparatus.
Fig. 2 illustrates an example of an ultrasound image.
Fig. 3 is a flowchart illustrating the procedure of a process of the ultrasound diagnostic apparatus.
Fig. 4 is a flowchart illustrating the procedure of the process of the ultrasound diagnostic apparatus.
Fig. 5 illustrates an example of a region of interest set in an ultrasound image.
Fig. 6 illustrates an aspect in which an ultrasound probe transmits ultrasonic waves.
Fig. 7 illustrates an aspect in which the ultrasound probe transmits the ultrasonic waves.
Fig. 8 illustrates an aspect in which the ultrasound probe transmits the ultrasonic waves.
Fig. 9 illustrates an aspect in which the ultrasound probe transmits the ultrasonic waves.
Fig. 10 illustrates an aspect in which the ultrasound probe transmits the ultrasonic waves.
Fig. 11 illustrates an aspect in which the ultrasound probe transmits the ultrasonic waves.
Fig. 12 illustrates an aspect in which the ultrasound probe transmits the ultrasonic waves.
Fig. 13 illustrates an aspect in which the ultrasound probe transmits the ultrasonic waves.
Fig. 14 illustrates an aspect in which the ultrasound probe transmits the ultrasonic waves.
Fig. 15 illustrates an aspect in which the ultrasound probe transmits the ultrasonic waves.
Fig. 16 illustrates an aspect in which the ultrasound probe transmits the ultrasonic waves.
Fig. 17 illustrates an aspect in which the ultrasound probe transmits the ultrasonic waves.
Fig. 18 is a flowchart illustrating the procedure of a process of determining the angle used.
Fig. 19 illustrates an aspect in which the ultrasound probe transmits the ultrasonic waves.

### DESCRIPTION OF THE PREFERRED EMBODIMENTS

In this embodiment, ultrasonic waves are used as acoustic waves. However, the acoustic waves are not limited to the ultrasonic waves and acoustic waves with audio frequencies may be used as long as appropriate frequencies can be selected according to, for example, subjects and measurement conditions.

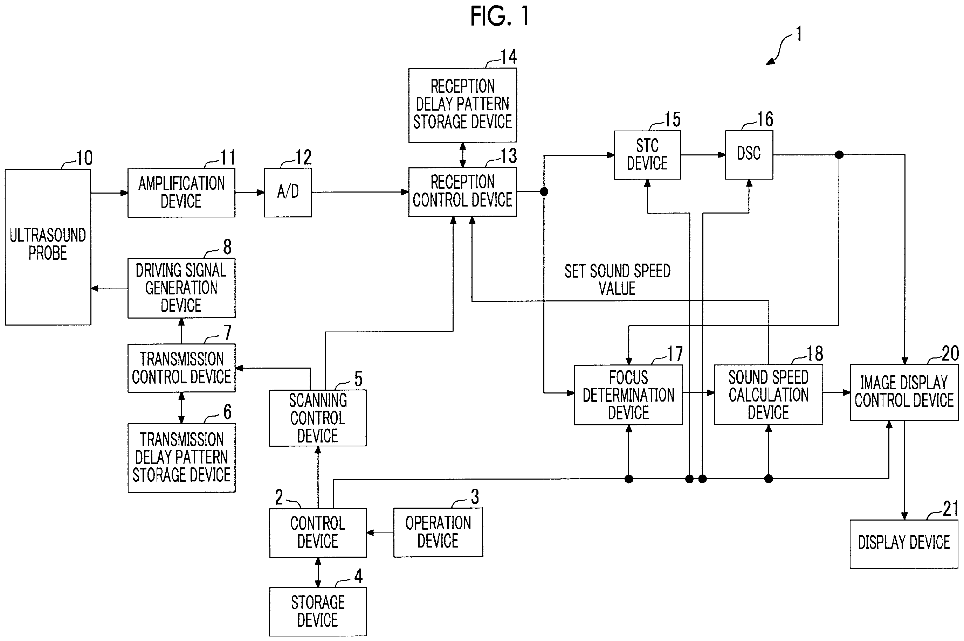

Fig. 1 illustrates an embodiment of the invention and is a block diagram illustrating the electric configuration of an ultrasound diagnostic apparatus 1 (sound speed calculation system).

The overall operation of the ultrasound diagnostic apparatus 1 is controlled by a control device 2.

An operation device 3 operated by a user (for example, a doctor, a nurse, or a medical technician) that operates the ultrasound diagnostic apparatus 1 and a storage device 4 that stores, for example, predetermined data are connected to the control device 2.

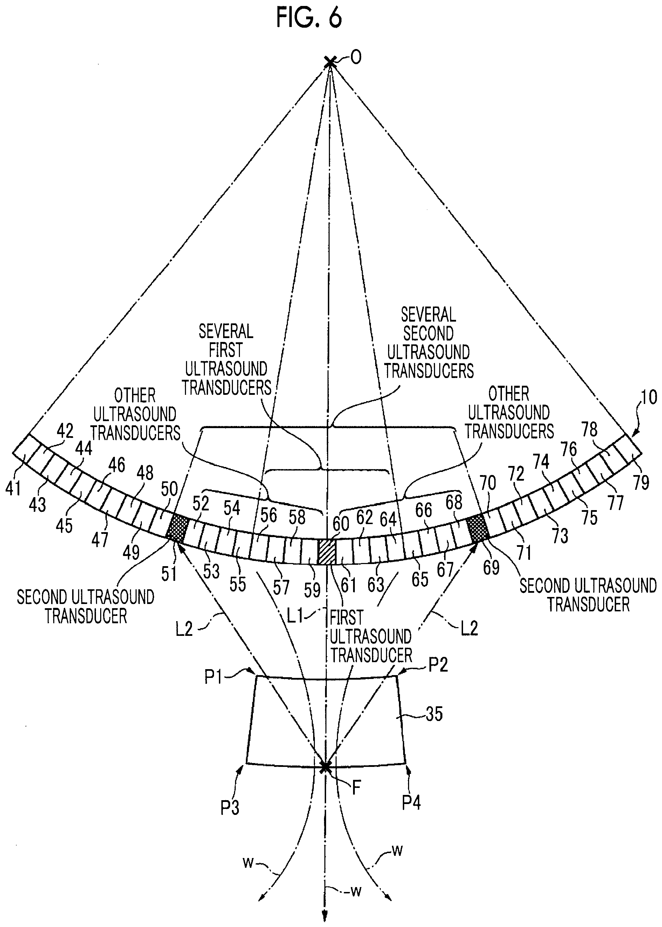

The ultrasound diagnostic apparatus 1 includes an ultrasound probe 10. The ultrasound probe 10 according to this embodiment is a convex type and includes a plurality of ultrasound transducers which are arranged in an arc shape (see, for example, Fig. 6).

A control signal that is output from the control device 2 is transmitted to a scanning control device 5. The scanning control device 5 sets an ultrasound transducer that transmits ultrasonic waves among a plurality of ultrasound transducers included in the ultrasound probe 10 and the transmission direction of ultrasonic waves from the ultrasound probe 10. The scanning control device 5 transmits a control signal for setting the ultrasound transducer that transmits ultrasonic waves and a control signal for setting the transmission direction of the ultrasonic waves to a transmission control device 7. In addition, the scanning control device 5 sets an ultrasound transducer that receives ultrasound echoes from a subject among the plurality of ultrasound transducers included in the ultrasound probe 10. The scanning control device 5 transmits a control signal for setting the ultrasound transducer that receives ultrasound echoes to a reception control device 13.

The ultrasound diagnostic apparatus 1 further includes a transmission delay pattern storage device 6. The transmission delay pattern storage device 6 stores a plurality of transmission delay patterns that are used in a case in which ultrasonic waves are transmitted from the ultrasound probe 10. The transmission control device 7 selects a transmission delay pattern from the plurality of transmission delay patterns stored in the transmission delay pattern storage device 6 on the basis of the control signal for setting the transmission direction of the ultrasonic waves which is transmitted from the scanning control device 5. The transmission control device 7 controls a driving signal generation device 8 such that ultrasonic waves are transmitted from the ultrasound probe 10 on the basis of the selected transmission delay pattern. The driving signal generation device 8 controls the ultrasound probe 10 such that ultrasonic waves are transmitted to the subject from the ultrasound transducer set by the scanning control device 5 among the plurality of ultrasound transducers included in the ultrasound probe 10. The control device 2, the scanning control device 5, the transmission delay pattern storage device 6, the transmission control device 7, and the driving signal generation device 8 are transmission driving means for driving the ultrasound transducers (acoustic transducers) included in the ultrasound probe 10 such that the ultrasound transducers transmit ultrasonic waves (acoustic waves) to the subject.

In a case in which ultrasonic waves are transmitted to the subject, ultrasound echoes are generated from the subject and are received by the plurality of ultrasound transducers included in the ultrasound probe 10. An ultrasound echo signal is output from the ultrasound transducer and is transmitted to an amplification device 11. The ultrasound echo signal is amplified by the amplification device 11 and is converted into digital ultrasound echo data by an analog/digital (A/D) conversion circuit 12. The ultrasound echo data is input to the reception control device 13.

The ultrasound diagnostic apparatus 1 further includes a reception delay pattern storage device 14. The reception delay pattern storage device 14 stores a plurality of reception delay patterns that are used in a case in which a reception focus process is performed for the ultrasound echo data. The reception control device 13 selects a reception delay pattern from the plurality of reception delay patterns stored in the reception delay pattern storage device 14 on the basis of the control signal transmitted from the scanning control device 5. The reception control device 13 performs the reception focus process by applying delay to a plurality of ultrasound echo data items on the basis of the selected reception delay pattern and a set sound speed value that is transmitted from a sound speed calculation device 18 to the reception control device 13 and adding the plurality of ultrasound echo data items. An envelope detection process is performed for the generated data by the reception focus process. In an initial state in which a sound speed has not been calculated, the set sound speed value transmitted from the sound speed calculation device 18 to the reception control device 23 is a sound speed CO (1530 m/s or 1540 m/s) in a general living body. Then, the calculated average sound speed Ci is used.

The amount of delay of the ultrasound echo data in the reception focus process is determined on the basis of the sound speed in the subject. In general, a sound speed of 1530m/s or 1540m/s is set as the sound speed C0 in the living body. In practice, the sound speed varies depending on tissues in the living body. Therefore, the average sound speed Ci in the subject is set and the amount of delay D0(j) in the reception delay pattern is multiplied by (C0/Ci) to determine a plurality of amounts of delay D1(j) = (C0/Ci)-D0(j) (j = 1, 2, ..., N). However, N is the number of ultrasound transducers used.

The data generated by the reception control device 13 is transmitted to a sensitivity time control (STC) device 15. The STC device 15 performs a process of correcting attenuation according to the depth of an ultrasound reflection position for the data transmitted from the reception control device 13. The data whose attenuation has been corrected by the STC device 15 is transmitted to a digital scan converter (DSC) 16. The DSC 16 performs raster conversion such that the attenuation-corrected sound ray data becomes image data based on a general television signal scanning system and performs necessary image processing, such as gradation processing, for the image data to generate B-mode image data. The generated B-mode image data is transmitted to a display device 21. The display device 21 displays the ultrasound image (B-mode image) of the subject on a display screen 30 of the display device 21. The STC device 15 and the DSC 16 are acoustic image generation means.

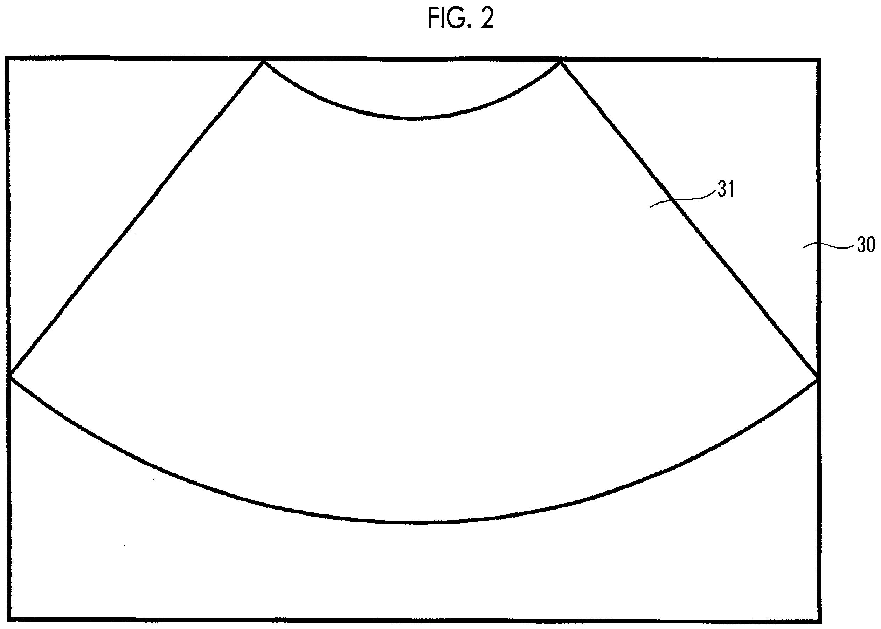

Fig. 2 illustrates an example of an ultrasound image 31 that is displayed on the display screen 30 of the display device 21.

Since the ultrasound probe 10 is a convex type, the obtained ultrasound image 31 has a shape surrounded by two arcs and two straight lines. The doctor sees the ultrasound image 31 and diagnoses the subject.

The control device 2 controls the sound speed calculation device 18 such that the sound speed calculation device 18 sequentially changes the set sound speed to the average sound speed Ci. A focus determination device 17 determines the degree of beam focusing in the reception focus process in a case in which the set sound speed is changed to the average sound speed Ci.

For example, the focus determination device 17 performs fast Fourier transform for the data generated by the reception control device 13 and determines the degree of beam focusing to be the maximum in a case in which the ratio of high-frequency components (for example, the ratio of high-frequency components to medium-frequency components) in the generated data is the maximum. In addition, the focus determination device 17 may perform fast Fourier transform for the B-mode image data output from the DSC and may determine the degree of beam focusing to be the maximum in a case in which the ratio of high-frequency components of a spatial frequency in the B-mode image data is the maximum.

In a case in which the focus determination device 17 determines that the degree of beam focusing is the maximum, a sound speed value calculation unit 42 divides the distance from the focus that is set in the subject to ultrasound echoes by the time for which the ultrasound transducers included in the ultrasound probe 10 receive the ultrasound echoes from the focus set in the subject to calculate the average sound speed Ci of the subject. The calculation of the sound speed will be described in detail below.

An image display control device 20 selects at least one of the generated B-mode image data or data indicating the average sound speed Ci calculated by the sound speed calculation device 18 and generates display image data, in response to the operation of the user through the operation device 3. The display device 21 displays the ultrasound image or the average sound speed Ci on the basis of the display image data. The display device 21 displays the ultrasound image and the average sound speed Ci.

Next, a method for calculating the sound speed (average sound speed Ci) in the subject will be described.

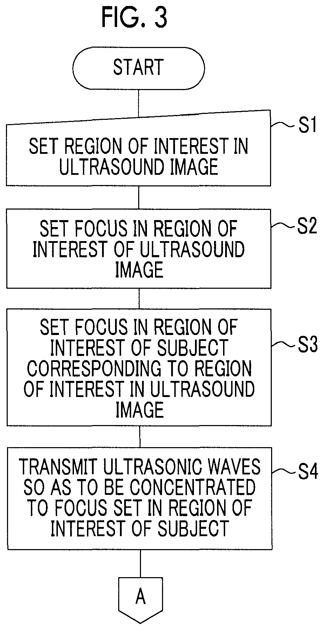

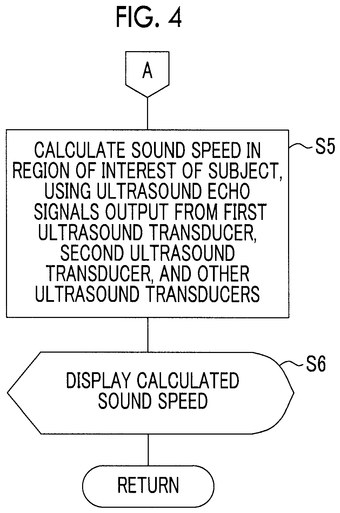

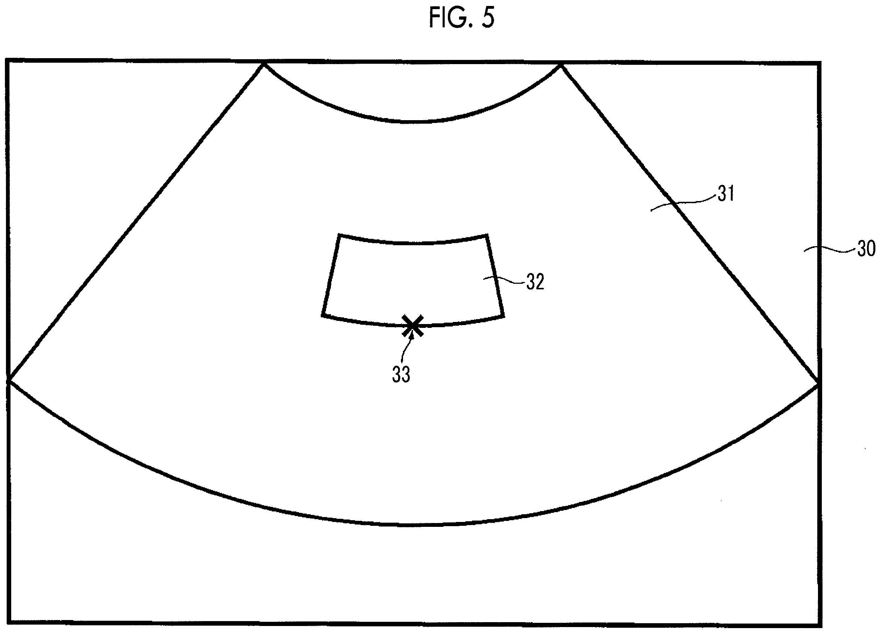

Figs. 3 and 4 are flowcharts illustrating the procedure of a sound speed calculation process and illustrates the procedure of the process of the control device 2. Fig. 5 illustrates an example of the ultrasound image 31 displayed on the display screen 30 of the display device 21.

In a case in which the ultrasound image 31 of the subject is displayed on the display screen 30 of the display device 21 as illustrated in Fig. 5, the user of the ultrasound diagnostic apparatus 1 sets a region of interest 32 in the ultrasound image 31, using the operation device 3 (region-of-interest setting means) (Step S1). The set region of interest 32 is displayed on the ultrasound image 31. Then, a focus 33 is set by the control device 2 (Step S2). The set focus 33 is also displayed on the ultrasound image 31. The focus 33 indicates the focal position of the ultrasonic waves transmitted from the ultrasound probe 10 to the subject. In this embodiment, the focus 33 is set in a portion that is farthest in the transmission direction of the ultrasonic waves in the region of interest 32 of the ultrasound image 31.

In a case in which the region of interest 32 and the focus 33 are set on the display screen 30 of the display device 21, the focus is set in a region of interest of the subject which corresponds to the region of interest 32 of the display screen 30.

Fig. 6 illustrates an aspect in which the ultrasound probe 10 transmits ultrasonic waves w to the subject.

The ultrasound probe 10 includes a plurality of ultrasound transducers 41 to 79 which are arranged in an arc shape. The ultrasound probe 10 transmits ultrasonic waves w to the subject in a direction from the center O of the arc to the opposite side of the ultrasound probe 10. Among the plurality of ultrasound transducers 40 to 79 included in the ultrasound probe 10, several first ultrasound transducers (acoustic transducers) 56 to 64 transmit ultrasonic waves to the subject (the several first ultrasound transducers mean ultrasound transducers that transmit ultrasonic waves and may be some of the ultrasound transducers included in the ultrasound probe 10, may be all of the ultrasound transducers 41 to 79, or may be the same as several second ultrasound transducers 51 to 69 which will be described below), or other ultrasound transducers may transmits ultrasonic waves to the subject. The control device 2 (focus setting means) sets a focus F in a portion that is farthest in the transmission direction of the ultrasonic waves in a region of interest 35 of the subject which corresponds to the region of interest 32 of the ultrasound image 31 set on the display screen 30. In the example illustrated in Fig. 6, the region of interest 35 of the subject has a shape surrounded by two radii and two arcs of a circle. A portion that is farthest in the transmission direction of the ultrasonic waves w is the center of the arc that is farthest from the ultrasound probe 10 in the region of interest 35. The center may not be the perfect center and may be a portion that is substantially regarded as the center. The focus F set in the subject corresponds to the focus 33 set on the display screen 30 of the display device 21. The focus F is the position to which the ultrasonic waves w transmitted from the several first ultrasound transducers 56 to 64 are concentrated.

In a case in which the driving signal generation device 8 transmits a driving signal to the ultrasound probe 10, the several first ultrasound transducers 56 to 64 transmit the ultrasonic waves w so as to be concentrated to the focus F (Step S4).

In this embodiment, among the plurality of ultrasound transducers 41 to 79 included in the ultrasound probe 10, the several second ultrasound transducers 51 to 69 receive the ultrasound echoes from the focus F. The several second ultrasound transducers 51 to 69 mean ultrasound transducers that receive the ultrasound echoes from the focus F. The several second ultrasound transducers 51 to 69 may be the same as the several first ultrasound transducers 56 to 64 or may be all of the plurality of ultrasound transducers 41 to 79 included in the ultrasound probe 10.

It is assumed that, among the several second ultrasound transducers 51 to 69 receiving the ultrasound echoes from the focus F, the ultrasound transducer 60 (in Fig. 6, a central ultrasound transducer among the plurality of ultrasound transducers 41 to 79) that is closest to the focus F is referred to as a first ultrasound transducer 60. In Fig. 6, the focus F is located on a first straight line L1 that is the transmission direction of the ultrasonic waves from the first ultrasound transducer 60. It is assumed that, among the several second ultrasound transducers 51 to 69, two ultrasound transducers (in Fig. 6, the ultrasound transducers 51 and 69) 51 and 69 that are other than the first ultrasound transducer 60, are located on straight lines L2 passing through the focus F and the region of interest 35 of the subject, and have the first ultrasound transducer 60 interposed therebetween are referred to as second ultrasound transducers. In addition, it is assumed that, among the several second ultrasound transducers 51 to 69, the ultrasound transducers 52 to 59 between the first ultrasound transducer 60 and the second ultrasound transducer 51 and the ultrasound transducers 61 to 68 between the first ultrasound transducer 60 and the second ultrasound transducer 69 are referred to as other ultrasound transducers.

In this embodiment, the sound speed calculation device 18 (sound speed calculation means) calculates the sound speed (average sound speed Ci) in the region of interest 35 of the subject, using the ultrasound echo signals output from the first ultrasound transducer 60, the two second ultrasound transducers 51 and 69, and other ultrasound transducers 52 to 59 and 61 to 69 among the several second ultrasound transducers 51 to 69. The ultrasound echo signals output from the first ultrasound transducer 60, the two second ultrasound transducers 51 and 69, and other ultrasound transducers 52 to 59 and 61 to 69 among the several second ultrasound transducers 51 to 69 are amplified by the amplification device 11 and are converted into digital ultrasound echo data by the A/D conversion circuit 12. The ultrasound echo data is transmitted to the reception control device 13. The reception control device 13 generates sound ray data from the ultrasound echo data. The sound ray data is input to the focus determination device 17. The focus determination device 17 performs determination. Data indicating the determination result of the focus determination device 17 is input to the sound speed calculation device 18 and the sound speed in the subject is calculated (Step S5).

The proportion of the paths of the ultrasound echoes through the region of interest 35 of the subject to the paths of the ultrasound echoes used to calculate the sound speed is high. Since the sound speed is calculated on the basis of the ultrasound echoes, the calculated sound speed indicates an accurate sound speed in the region of interest 35 of the subject. The calculated sound speed is displayed on the display screen 30 of the display device 21 under the control of the image display control device 20 (Step S6).

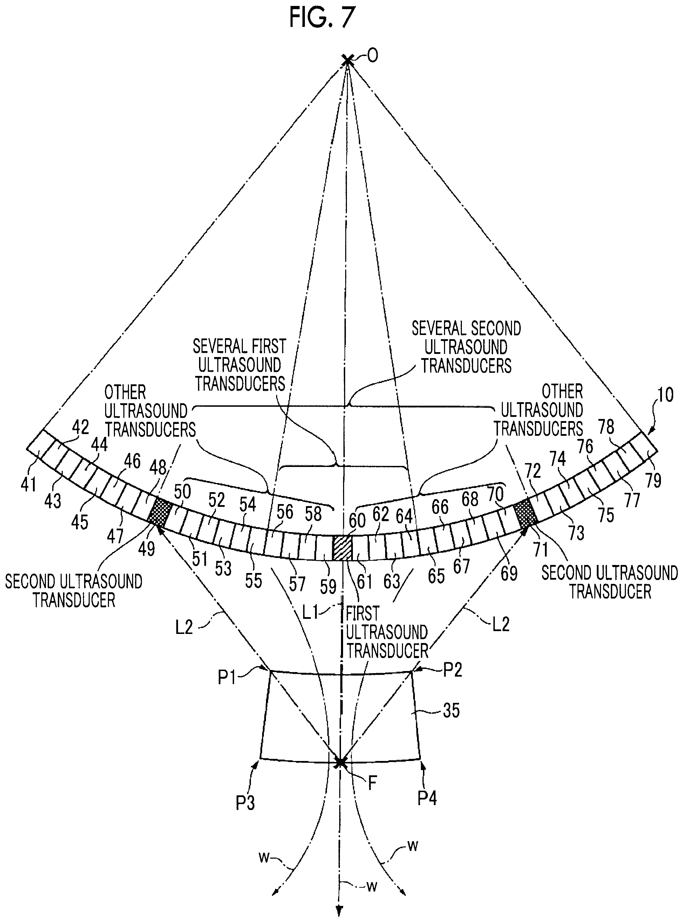

Fig. 7 illustrates another embodiment and illustrates an aspect in which the ultrasound probe 10 transmits the ultrasonic waves w to the subject.

The region of interest 35 of the subject is set so as to correspond to the region of interest 32 of the display screen 30. The focus F is set in a portion that is farthest in the transmission direction in the region of interest 35 of the subject.

A straight line connecting the focus F and the first ultrasound transducer 60 that is closest to the focus F is the first straight line L1. It is assumed that straight lines connecting the focus F and two first vertexes P1 and P2 which are farthest from the focus F among four vertexes P1, P2, P3, and P4 of the region of interest 35 of the subject are the second straight lines L2. In this case, the control device 2 (sound speed calculation means) sets the second ultrasound transducers to the ultrasound transducers 49 and 71 that are located on the second straight lines L2.

The several first ultrasound transducers 56 to 64 transmit the ultrasonic waves w and the several second ultrasound transducers 49 to 71 receive ultrasound echoes from the focus F. The sound speed calculation device 18 calculates the sound speed in the region of interest 35 of the subject, using the ultrasound echo signals output from the first ultrasound transducer 60, the two second ultrasound transducers 49 and 71, other ultrasound transducers 50 to 59 between one second ultrasound transducer 49 of the two second ultrasound transducer 49 and 71 and the first ultrasound transducer 60 and other ultrasound transducers 61 to 70 between the first ultrasound transducer 60 and the other second ultrasound transducer 71.

The proportion of the paths of the ultrasound echoes through the region of interest 35 of the subject to the paths of the ultrasound echoes from the focus F to the several second ultrasound transducers 51 to 69 is high. Therefore, it is possible to accurately calculate the sound speed in the region of interest 35 of the subject.

The control device 2 (sound speed calculation means) does not necessarily clearly set the second ultrasound transducers to the ultrasound transducers 51 and 69 located on the second straight lines L2. The sound speed in the region of interest 35 of the subject may be calculated, using the ultrasound echo signals output from the ultrasound transducers 51 to 69 in the range interposed between two second straight lines L2 passing through the focus F and two vertexes P1 and P2 that are farthest from the focus F among the vertexes P1, P2, P3, and P4 of the region of interest 35 of the subject.

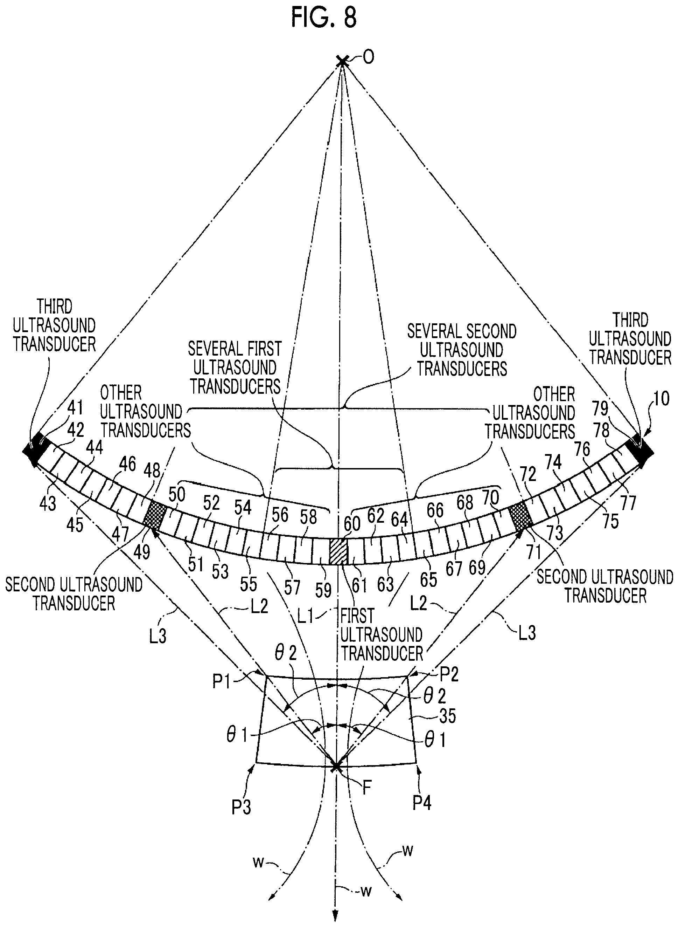

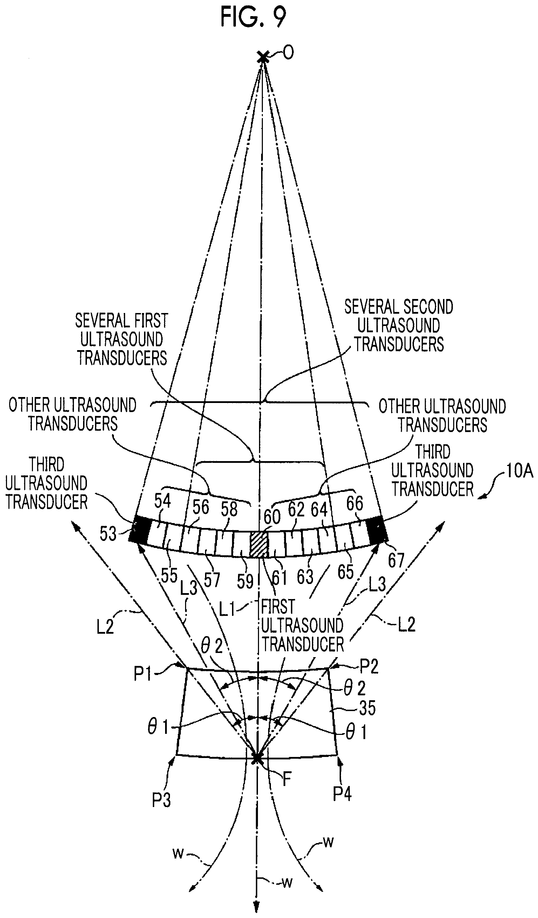

Figs. 8 and 9 illustrate another embodiment and illustrate an aspect in which the ultrasound probe 10 transmits the ultrasonic waves w to the subject.

In Figs. 8 and 9, the region of interest 35 of the subject is set so as to correspond to the region of interest 32 of the display screen 30. The focus F is set in a portion which is farthest in the transmission direction in the region of interest 35 of the subject.

A straight line connecting the focus F and the first ultrasound transducer 60 that is closest to the focus F is the first straight line L1. Straight lines connecting the focus F and two first vertexes P1 and P2 which are farthest from the focus F among four vertexes P1, P2, P3, and P4 of the region of interest 35 of the subject are the second straight lines L2. In addition, it is assumed that, among the plurality of ultrasound transducers 41 to 79 included in the ultrasound probe 10, an ultrasound transducer that is farthest from the focus F is a third ultrasound transducer and a straight line connecting the focus F and the third ultrasound transducer is a third straight line L3.

In the case illustrated in Fig. 8, among the plurality of ultrasound transducers 41 to 79, the ultrasound transducers that are farthest from the focus F are the ultrasound transducers 41 and 79. The ultrasound transducers 41 and 79 are the third ultrasound transducers. Straight lines connecting the focus F and the ultrasound transducers 41 and 79 are the third straight lines L3.

A first angle θ1 formed between the first straight line L1 and the second straight line L2 is less than a second angle θ2 formed between the first straight line L1 and the third straight line L3. As such, in a case in which the first angle θ1 formed between the first straight line L1 and the second straight line L2 is less than the second angle θ2 formed between the first straight line L1 and the third straight line L3, the second ultrasound transducers are set to the ultrasound transducers 49 and 71 located on the second straight lines L2. The sound speed calculation device 18 calculates the sound speed, using the ultrasound echo signals output from the first ultrasound transducer 60, the second ultrasound transducers 49 and 71, other ultrasound transducers 50 to 59 between the first ultrasound transducer 60 and the second ultrasound transducer 49, and other ultrasound transducers 61 to 70 between the first ultrasound transducer 60 and the second ultrasound transducer 71.

An ultrasound probe 10A illustrated in Fig. 9 includes a plurality of ultrasound transducers 53 to 67 whose number is less than the number of ultrasound transducers 41 to 79 included in the ultrasound probe 10B illustrated in Fig. 8.

Among the plurality of ultrasound transducers 53 to 67, ultrasound transducers that are farthest from the focus F are the ultrasound transducers 53 and 67. The ultrasound transducers 53 and 67 are the third ultrasound transducers. Straight lines connecting the focus F and the ultrasound transducers 53 and 67 are the third straight lines L3.

In the case illustrated in Fig. 9, the first angle θ1 formed between the first straight line L1 and the second straight line L2 is greater than the second angle θ2 formed between the first straight line L1 and the third straight line L3. As such, in a case in which the first angle θ1 formed between the first straight line L1 and the second straight line L2 is greater than the second angle θ2 formed between the first straight line L1 and the third straight line L3, the second ultrasound transducers are set to the third ultrasound transducers 53 and 67 and the sound speed calculation device 18 calculates the sound speed, using the ultrasound echo signals output from the first ultrasound transducer 60, the set second ultrasound transducers 53 and 67, other ultrasound transducers 54 to 59 between the first ultrasound transducer 60 and the second ultrasound transducer 53, and other ultrasound transducers 61 to 66 between the first ultrasound transducer 60 and the second ultrasound transducer 67.

In the embodiment illustrated in Fig. 8, the control device 2 (sound speed calculation means) does not necessarily clearly set the ultrasound transducers 49 and 71 located on the second straight lines L2 to the second ultrasound transducers and does not necessarily clearly set the ultrasound transducers 41 and 79 located on the third straight lines L3 to the third ultrasound transducers. In a case in which the first angle θ1 formed between the first straight line L1 and the second straight line L2 is less than the second angle θ2 formed between the first straight line L1 and the third straight line L3, the sound speed in the region of interest 35 of the subject may be calculated using the ultrasound echo signals output from the ultrasound transducers 49 to 71 in the range interposed between two second straight lines L2 (the range included in the first angle θ1 between the first straight line L1 and the second straight line L2).

In the embodiment illustrated in Fig. 9, the control device 2 (sound speed calculation means) does not necessarily clearly set the second ultrasound transducers to the third ultrasound transducers. In a case in which the first angle θ1 formed between the first straight line L1 and the second straight line L2 is greater than the second angle θ2 formed between the first straight line L1 and the third straight line L3, the sound speed in the region of interest 35 of the subject may be calculated using the ultrasound echo signals output from the ultrasound transducers 53 to 67 in the range interposed between two third straight lines L3 (the range included in the second angle θ2 between the first straight line L1 and the third straight line L3).

In a case in which the first angle θ1 formed between the first straight line L1 and the second straight line L2 is equal to the second angle θ2 formed between the first straight line L1 and the third straight line L3, the sound speed in the region of interest 35 of the subject may be calculated as illustrated in Fig. 8 or the sound speed in the region of interest 35 of the subject may be calculated as illustrated in Fig. 9. Furthermore, in a case in which the first angle θ1 formed between the first straight line L1 and the second straight line L2 is equal to the second angle θ2 formed between the first straight line L1 and the third straight line L3, the sound speed in the region of interest 35 of the subject may be calculated, using the ultrasound echo signals output from the ultrasound transducers in the range interposed between two second straight lines L2 (the range included in the first angle θ1 formed between the first straight line L1 and the second straight line L2) or the range interposed between two third straight lines L3 (the range included in the second angle θ2 formed between the first straight line L1 and the third straight line L3).

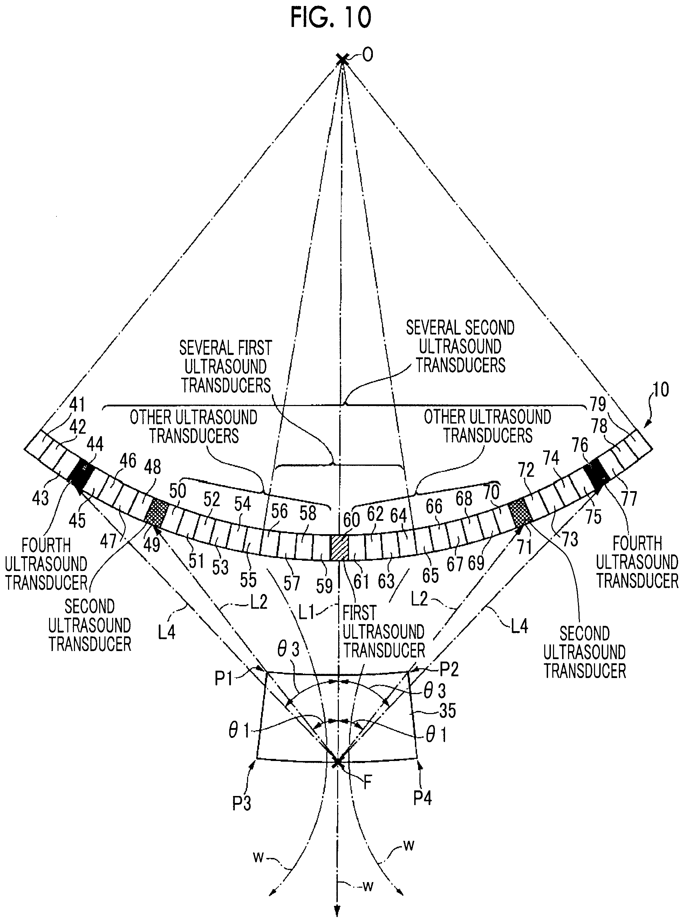

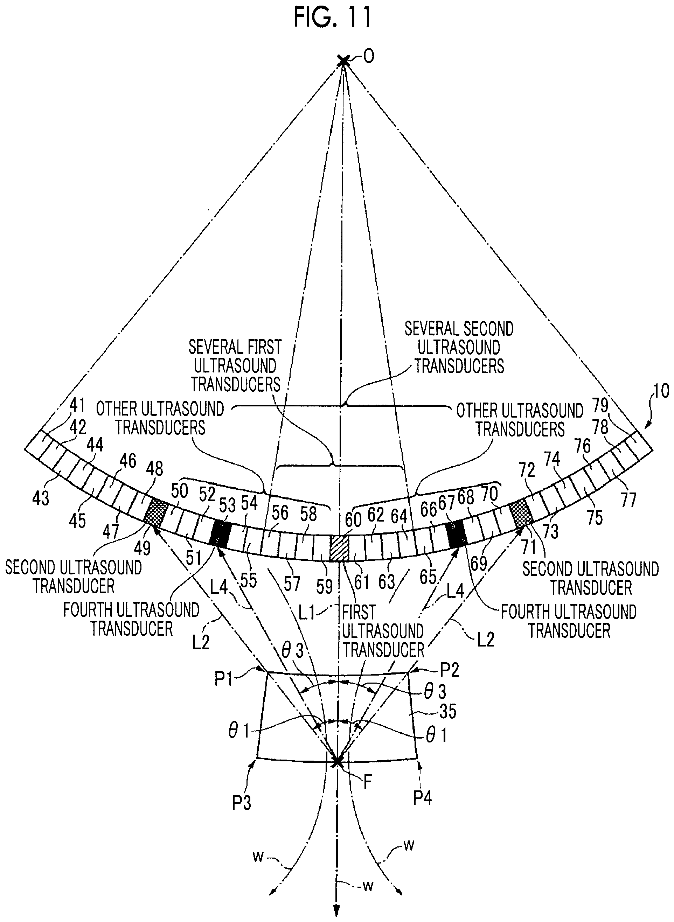

Figs. 10 and 11 illustrate another embodiment and illustrate an aspect in which the ultrasound probe 10 transmits the ultrasonic waves w to the subject.

In Figs. 10 and 11, the region of interest 35 of the subject is set so as to correspond to the region of interest 32 of the display screen 30. The focus F is set in a portion that is farthest in the transmission direction in the region of interest 35 of the subject.

In Figs. 10 and 11, a straight line connecting the focus F and the first ultrasound transducer 60 that is closest to the focus F is the first straight line L1. Straight lines connecting the focus F and two first vertexes P1 and P2 which are farthest from the focus F among four vertexes P1, P2, P3, and P4 of the region of interest 35 of the subject are the second straight lines L2.

In the ultrasound probe 10 illustrated in Figs. 10 and 11, among a plurality of ultrasound transducers 41 to 79 included in the ultrasound probe 10, the maximum number of ultrasound transducers that can receive the ultrasound echoes used to calculate the sound speed in the region of interest 35 of the subject at the same time is the several second ultrasound transducers. In addition, it is assumed that an ultrasound transducer that is farthest from the focus F among the several second ultrasound transducers is a fourth ultrasound transducer. It is assumed that a straight line connecting the focus F and the fourth ultrasound transducer is a fourth straight line L4.

In Fig. 10, the maximum number of ultrasound transducers that can receive the ultrasound echoes used to calculate the sound speed in the region of interest 35 of the subject at the same time is the ultrasound transducers 44 to 76. The fourth ultrasound transducers that are farthest from the focus F among the several second ultrasound transducers 44 to 76 are the ultrasound transducers 44 and 76.

In Fig. 10, the first angle θ1 formed between the first straight line L1 and the second straight line L2 is less than a third angle θ3 formed between the first straight line L1 and the fourth straight line L4. As such, in a case in which the first angle θ1 formed between the first straight line L1 and the second straight line L2 is less than the third angle θ3 formed between the first straight line L1 and the fourth straight line L4, the control device 2 (sound speed calculation means) sets the second ultrasound transducers to the ultrasound transducers 49 and 71 located on the second straight lines L2. The sound speed calculation device 18 calculates the sound speed in the region of interest 35 of the subject, using the ultrasound echo signals output from the first ultrasound transducer 60, the second ultrasound transducers 49 and 71, other ultrasound transducers 50 to 59 between the second ultrasound transducer 49 and the first ultrasound transducer 60, and other ultrasound transducers 61 to 70 between the second ultrasound transducer 71 and the first ultrasound transducer 60.

In the embodiment illustrated in Fig. 10, the control device 2 (sound speed calculation means) does not necessarily clearly set the second ultrasound transducers to the ultrasound transducers 49 and 71 located on the second straight lines L2. In a case in which the first angle θ1 formed between the first straight line L1 and the second straight line L2 is less than the third angle θ3 formed between the first straight line L1 and the fourth straight line L4, the sound speed in the region of interest 35 of the subject may be calculated using the ultrasound echo signals output from the ultrasound transducers 49 to 71 in the range interposed between two second straight lines L2 (the range included in the first angle θ1 formed between the first straight line L1 and the second straight line L2).

In Fig. 11, the maximum number of ultrasound transducers that can receive the ultrasound echoes used to calculate the sound speed in the region of interest 35 of the subject at the same time is the ultrasound transducers 53 to 67. The several second ultrasound transducers are the ultrasound transducers 53 to 67. The fourth ultrasound transducers that are farthest from the focus F among the several second ultrasound transducers 53 to 67 are the ultrasound transducers 53 and 67.

In Fig. 11, the first angle θ1 formed between the first straight line L1 and the second straight line L2 is greater than the third angle θ3 formed between the first straight line L1 and the fourth straight line L4. As such, in a case in which the first angle θ1 formed between the first straight line L1 and the second straight line L2 is greater than the third angle θ3 formed between the first straight line L1 and the fourth straight line L4, the control device 2 (sound speed calculation means) sets the second ultrasound transducers 53 and 71 to the fourth ultrasound transducers 53 and 67. The sound speed calculation device 18 calculates the sound speed in the region of interest 35 of the subject, using the ultrasound echo signals output from the first ultrasound transducer 60, the second ultrasound transducers 49 and 71, other ultrasound transducers 54 to 59 between the fourth ultrasound transducer 53 set as the second ultrasound transducer and the first ultrasound transducer 60, and other ultrasound transducers 61 to 66 between the fourth ultrasound transducer 67 and the first ultrasound transducer 60.

In the embodiment illustrated in Fig. 11, the control device 2 (sound speed calculation means) does not necessarily clearly set the second ultrasound transducers to the fourth ultrasound transducers 53 and 67. In a case in which the first angle θ1 formed between the first straight line L1 and the second straight line L2 is greater than the third angle θ3 formed between the first straight line L1 and the fourth straight line L4, the sound speed in the region of interest 35 of the subject may be calculated using the ultrasound echo signals output from the ultrasound transducers 53 to 67 in the range interposed between two fourth straight lines L4 (the range included in the third angle θ3 formed between the first straight line L1 and the first straight line L1).

In a case in which the first angle θ1 formed between the first straight line L1 and the second straight line L2 is equal to the third angle θ3 formed between the first straight line L1 and the fourth straight line L4, the sound speed in the region of interest 35 of the subject may be calculated as illustrated in Fig. 10 or the sound speed in the region of interest 35 of the subject may be calculated as illustrated in Fig. 11.

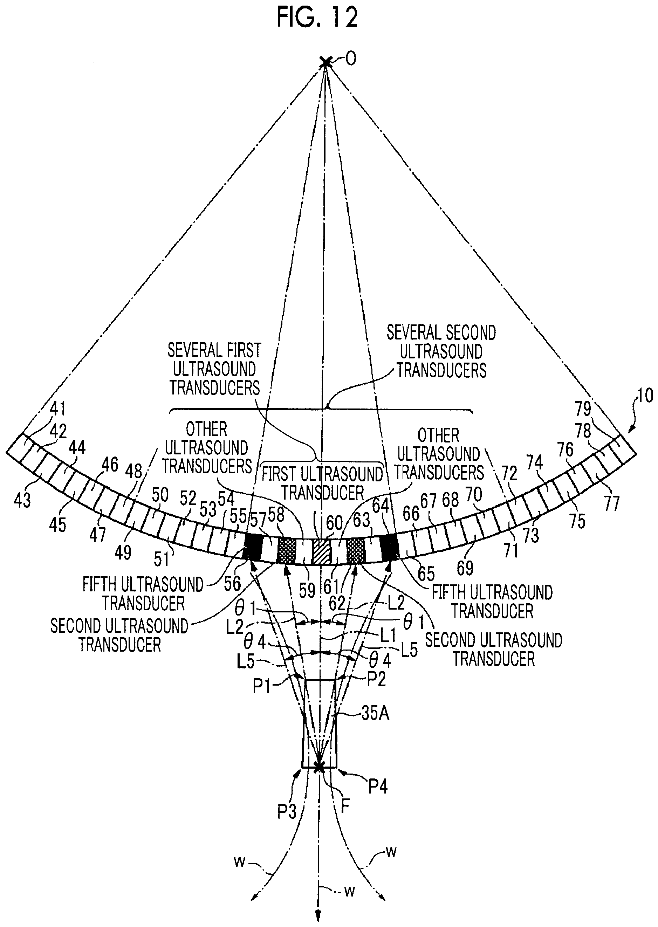

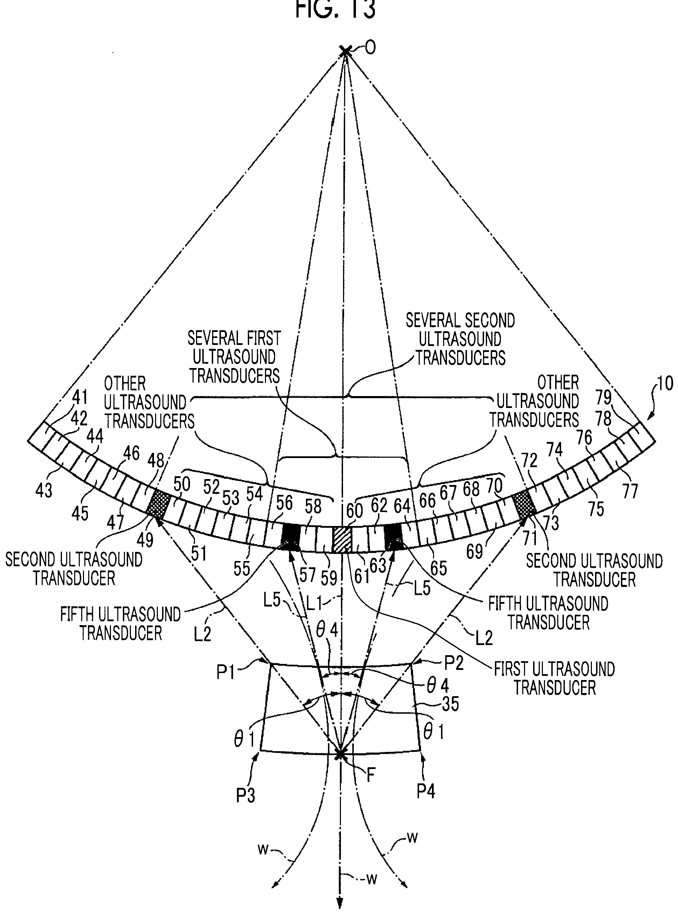

Figs. 12 and 13 illustrate another embodiment and illustrate an aspect in which the ultrasound probe 10 transmits the ultrasonic waves w to the subject.

In Figs. 12 and 13, the focus F is set in a portion that is farthest in the transmission direction in the region of interest 35 of the subject.

In Figs. 12 and 13, a straight line connecting the focus F and the first ultrasound transducer 60 that is closest to the focus F is the first straight line L1. In addition, straight lines connecting the focus F and two first vertexes P1 and P2 which are farthest from the focus F among four vertexes P1, P2, P3, and P4 of the region of interest 35 of the subject are the second straight lines L2.

In the ultrasound probe 10 illustrated in Figs. 12 and 13, fifth ultrasound transducers included in the several second ultrasound transducers are set. The fifth ultrasound transducer is configured such that a difference between a first distance between the focus F and the first ultrasound transducer 60 and a second distance between the focus F and the fifth ultrasound transducer is closest to a predetermined detection limit width. It is assumed that a straight line connecting the focus F and the fifth ultrasound transducer is a fifth straight line L5.

In Fig. 12, a region of interest 35A of the subject is set such that the length in the transmission direction of the ultrasonic waves w is greater than the length in the arc direction of the ultrasound probe 10.

The first angle θ1 formed between the first straight line L1 and the second straight line L2 is less than a fourth angle θ4 formed between the first straight line L1 and the fifth straight line L5. In a case in which the first angle θ1 formed between the first straight line L1 and the second straight line L2 is less than the fourth angle θ4 formed between the first straight line L1 and the fifth straight line L5, the second ultrasound transducers are set to the fifth ultrasound transducers 56 and 64. The sound speed calculation device 18 calculates the sound speed in the region of interest 35A of the subject, using the ultrasound echo signals output from the first ultrasound transducer 60, the second ultrasound transducers 56 and 64, other ultrasound transducers 57 to 59 between the second ultrasound transducer 56 and the first ultrasound transducer 60, and other ultrasound transducers 61 to 63 between the second ultrasound transducer 64 and the first ultrasound transducer 60.

In Fig. 12, the control device 2 (sound speed calculation means) does not necessarily clearly set the fifth ultrasound transducers to the ultrasound transducers 58 and 62 located on the second straight lines L2. In a case in which the first angle θ1 formed between the first straight line L1 and the second straight line L2 is less than the fourth angle θ4 formed between the first straight line L1 and the fifth straight line L5, the sound speed in the region of interest 35A of the subject may be calculated using the ultrasound echo signals output from the ultrasound transducers 56 to 64 in the range interposed between two fifth straight lines L5 (the range included in the fourth angle θ4 formed between the first straight line L1 and the fifth straight line L5).

In Fig. 13, the region of interest 35 of the subject is set so as to correspond to the region of interest 32 of the ultrasound image 31.

The first angle θ1 formed between the first straight line L1 and the second straight line L2 is greater than the fourth angle θ4 formed between the first straight line L1 and the fifth straight line L5. In a case in which the first angle θ1 formed between the first straight line L1 and the second straight line L2 is greater than the fourth angle θ4 formed between the first straight line L1 and the fifth straight line L5, the second ultrasound transducers are set to the ultrasound transducers 58 and 62 located on the second straight lines L2. The sound speed calculation device 18 calculates the sound speed in the region of interest 35 of the subject, using the ultrasound echo signals output from the first ultrasound transducer 60, the set second ultrasound transducers 58 and 62, another ultrasound transducer 59 between the second ultrasound transducer 58 and the first ultrasound transducer 60, and another ultrasound transducer 61 between the second ultrasound transducer 62 and the first ultrasound transducer 60.

In Fig. 13, the control device 2 (sound speed calculation means) does not necessarily clearly set the second ultrasound transducers to the fifth ultrasound transducers. In a case in which the first angle θ1 formed between the first straight line L1 and the second straight line L2 is greater than the fourth angle θ4 formed between the first straight line L1 and the fifth straight line L5, the sound speed in the region of interest 35 of the subject may be calculated using the ultrasound echo signals output from the ultrasound transducers 57 to 63 in the range interposed between two second straight lines L2 (the range included in the first angle θ1 formed between the first straight line L1 and the second straight line L2).

In a case in which the first angle θ1 formed between the first straight line L1 and the second straight line L2 is equal to the fourth angle θ4 formed between the first straight line L1 and the fifth straight line L5, the sound speed in the region of interest 35A of the subject may be calculated as illustrated in Fig. 12 or the sound speed in the region of interest 35 of the subject may be calculated as illustrated in Fig. 13.

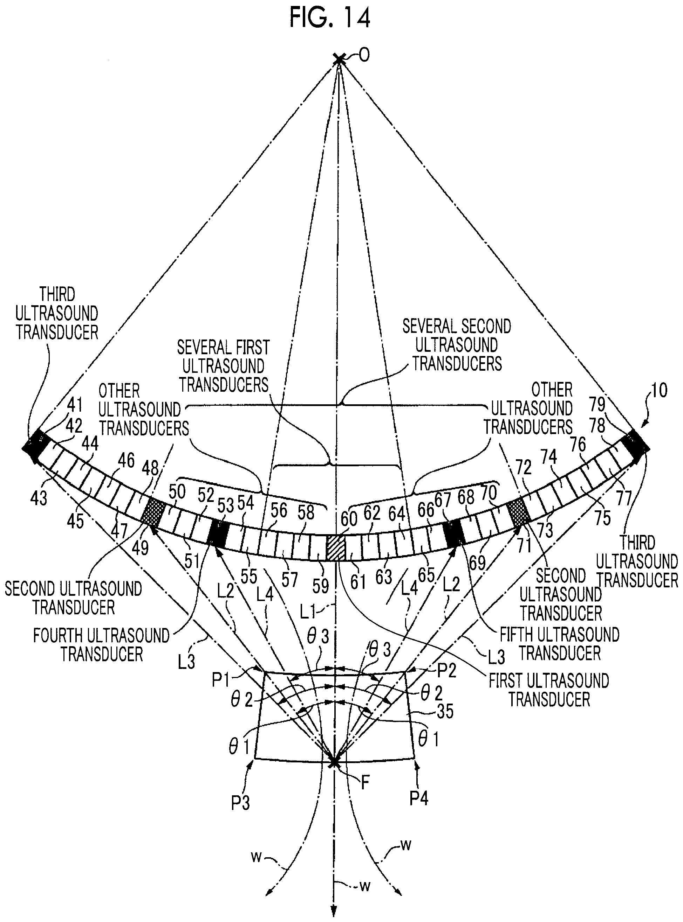

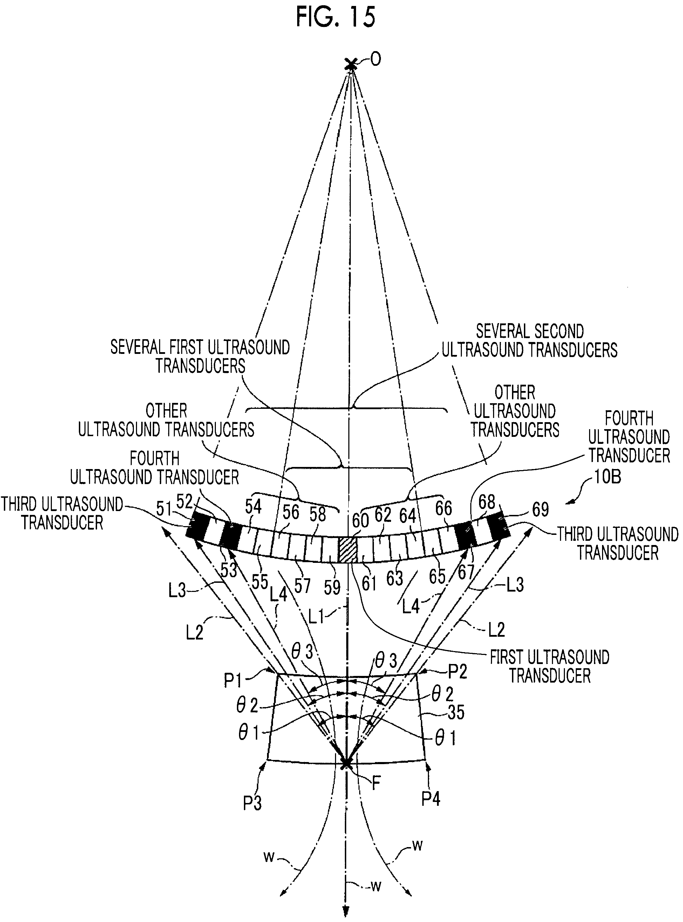

Figs. 14 and 15 illustrate another embodiment and illustrate an aspect in which the ultrasound probe 10 transmits the ultrasonic waves w to the subject.

In Figs. 14 and 15, the region of interest 35 of the subject is set so as to correspond to the region of interest 32 of the display screen 30. The focus F is set in a portion that is farthest in the transmission direction in the region of interest 35 of the subject.

In Figs. 14 and 15, a straight line connecting the focus F and the first ultrasound transducer 60 that is closest to the focus F is the first straight line L1. In addition, straight lines connecting the focus F and two first vertexes P1 and P2 which are farthest from the focus F among four vertexes P1, P2, P3, and P4 of the region of interest 35 of the subject are the second straight lines L2.

In the ultrasound probe 10 illustrated in Figs. 14 and 15, the maximum number of ultrasound transducers that can receive the ultrasound echoes used to calculate the sound speed in the region of interest 35 of the subject at the same time is the several second ultrasound transducers. In addition, it is assumed that the ultrasound transducers 41 and 79 that are farthest from the focus F among a plurality of ultrasound transducers 41 to 79 are the third ultrasound transducers. It is assumed that straight lines connecting the focus F and the third ultrasound transducers 41 and 79 are the third straight lines L3. In addition, it is assumed that an ultrasound transducer that is farthest from the focus F among the several second ultrasound transducers is the fourth ultrasound transducer. It is assumed that a straight line connecting the focus F and the fourth ultrasound transducer is the fourth straight line L4.

The first angle θ1 formed between the first straight line L1 and the second straight line L2 is greater than the third angle θ3 formed between the first straight line L1 and the fourth straight line L4.

In Fig. 14, the first angle θ1 formed between the first straight line L1 and the second straight line L2 is less than the second angle θ2 formed between the first straight line L1 and the third straight line L3. As such, in a case in which the first angle θ1 formed between the first straight line L1 and the second straight line L2 is less than the second angle θ2 formed between the first straight line L1 and the third straight line L3, the second ultrasound transducers are set to the fourth ultrasound transducer 53 and 67. The sound speed calculation device 18 calculates the sound speed in the region of interest 35 of the subject, using the ultrasound echo signals output from the first ultrasound transducer 60, the second ultrasound transducers 53 and 67, other ultrasound transducers 54 to 59 between the second ultrasound transducer 53 and the first ultrasound transducer 60, and other ultrasound transducers 61 to 66 between the second ultrasound transducer 67 and the first ultrasound transducer 60.

In Fig. 14, the control device 2 (sound speed calculation means) does not necessarily clearly set the second ultrasound transducers to the fourth ultrasound transducers. In a case in which the first angle θ1 formed between the first straight line L1 and the second straight line L2 is greater than the third angle θ3 formed between the first straight line L1 and the fourth straight line L4 and the first angle θ1 is less than the second angle θ2 formed between the first straight line L1 and the third straight line L3, the sound speed in the region of interest 35 of the subject may be calculated using the ultrasound echo signals output from the ultrasound transducers 53 to 67 in the range interposed between two fourth straight lines L4 (the range included in the third angle θ3 formed between the first straight line L1 and the fourth straight line L4).

In Fig. 15, an ultrasound probe 10B includes a plurality of ultrasound transducers 51 to 69.

The first angle θ1 formed between the first straight line L1 and the second straight line L2 is greater than the second angle θ2 formed between the first straight line L1 and the third straight line L3. As such, in a case in which the first angle θ1 formed between the first straight line L1 and the second straight line L2 is greater than the second angle θ2 formed between the first straight line L1 and the third straight line L3, the second ultrasound transducers are set to the third ultrasound transducers 51 and 69. The sound speed calculation device 18 calculates the sound speed in the region of interest 35 of the subject, using the ultrasound echo signals output from the first ultrasound transducer 60, the second ultrasound transducers 51 and 69, other ultrasound transducers 52 to 59 between the second ultrasound transducer 51 and the first ultrasound transducer 60, and other ultrasound transducers 61 to 68 between the first ultrasound transducer 60 and the second ultrasound transducer 69.

In a case in which the first angle θ1 is equal to the second angle 02, the sound speed in the region of interest 35 of the subject may be calculated as illustrated in Fig. 14 or the sound speed in the region of interest 35 of the subject may be calculated as illustrated in Fig. 15.

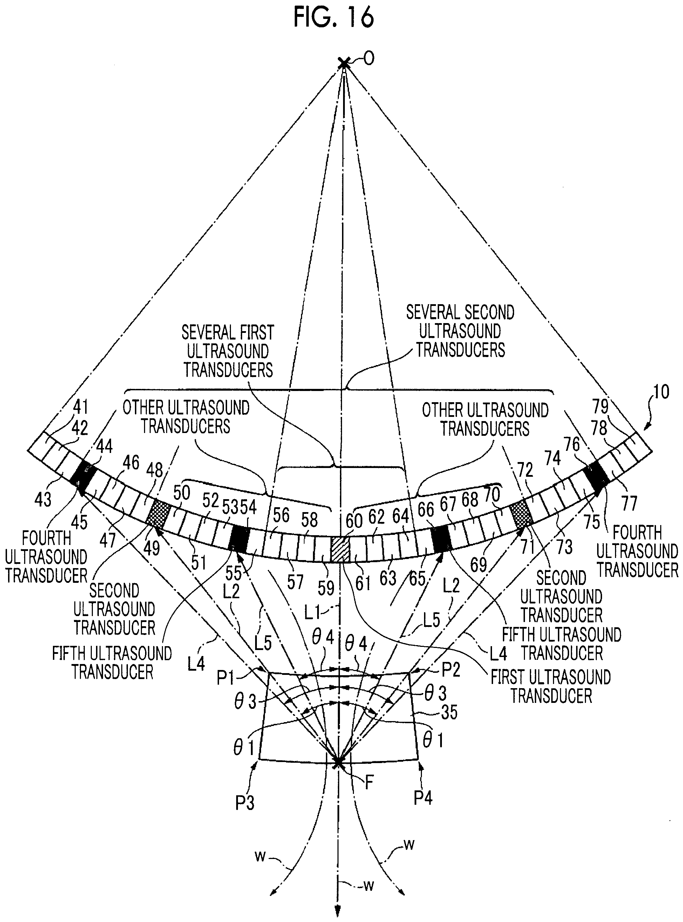

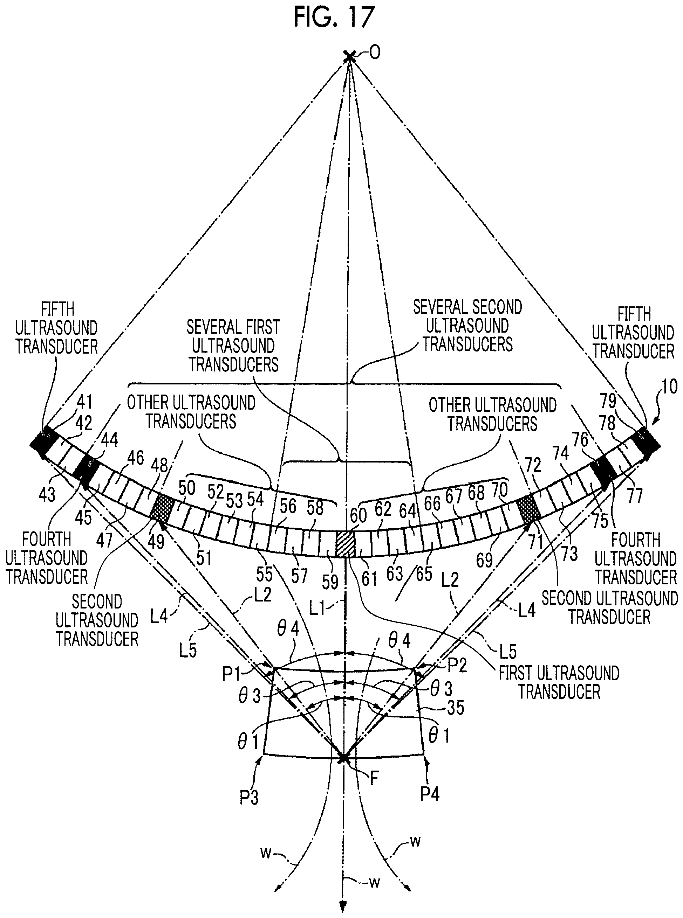

Figs. 16 and 17 illustrate still another embodiment and illustrate an aspect in which the ultrasound probe 10 transmits the ultrasonic waves w to the subject.

In Figs. 16 and 17, the region of interest 35 of the subject is set so as to correspond to the region of interest 32 of the display screen 30. The focus F is set in a portion that is farthest in the transmission direction in the region of interest 35 of the subject.

In Figs. 16 and 17, a straight line connecting the focus F and the first ultrasound transducer 60 that is closest to the focus F is the first straight line L1. In addition, straight lines connecting the focus F and two first vertexes P1 and P2 which are farthest from the focus F among four vertexes P1, P2, P3, and P4 of the region of interest 35 of the subject are the second straight lines L2.

In the ultrasound probe 10 illustrated in Figs. 16 and 17, the maximum number of ultrasound transducers that can receive the ultrasound echoes used to calculate the sound speed in the region of interest 35 of the subject at the same time is the several second ultrasound transducers. In addition, it is assumed that the ultrasound transducers 41 and 79 that are farthest from the focus F among a plurality of ultrasound transducers 41 to 79 are the third ultrasound transducers. It is assumed that straight lines connecting the focus F and the third ultrasound transducers 41 and 79 are the third straight lines L3. In addition, it is assumed that an ultrasound transducer that is farthest from the focus F among the several second ultrasound transducers is the fourth ultrasound transducer. It is assumed that a straight line connecting the focus F and the fourth ultrasound transducer is the fourth straight line L4.

For the fifth ultrasound transducer included in the several second ultrasound transducers, it is defined that the difference between the first distance between the focus F and the first ultrasound transducer 60 and the second distance between the focus F and the fifth ultrasound transducer is closest to a predetermined detection limit width. It is assumed that a straight line connecting the focus F and the fourth ultrasound transducer which is farthest from the focus F among the several second ultrasound transducers is the fifth straight line L5.

The first angle θ1 formed between the first straight line L1 and the second straight line L2 is less than the third angle θ3 formed between the first straight line L1 and the fourth straight line L4.

In Fig. 16, the first angle θ1 formed between the first straight line L1 and the second straight line L2 is greater than the fourth angle θ4 formed between the first straight line L1 and the fifth straight line L5. As such, in a case in which the first angle θ1 formed between the first straight line L1 and the second straight line L2 is greater than the fourth angle θ4 formed between the first straight line L1 and the fifth straight line L5, the second ultrasound transducers are set to the ultrasound transducers 49 and 71 located on the second straight lines L2. The sound speed calculation device 18 calculates the sound speed in the region of interest 35 of the subject, using the ultrasound echo signals output from the first ultrasound transducer 60, the second ultrasound transducers 49 and 71, other ultrasound transducers 50 to 70 between the second ultrasound transducer 49 and the first ultrasound transducer 60, and other ultrasound transducers 61 to 70 between the first ultrasound transducer 60 and the second ultrasound transducer 71.

In Fig. 16, the control device 2 (sound speed calculation means) does not necessarily clearly set the second ultrasound transducers to the ultrasound transducers located on the second straight lines L2. In a case in which the first angle θ1 is less than the third angle θ3 and the first angle θ1 is greater than the fourth angle θ4, the sound speed in the region of interest 35 of the subject may be calculated using the ultrasound echo signals output from the ultrasound transducers 49 to 71 in the range interposed between two second straight lines L2 (the range included in the first angle θ1 formed between the first straight line L1 and the second straight line L2).

In Fig. 17, the first angle θ1 formed between the first straight line L1 and the second straight line L2 is less than the fourth angle θ4 formed between the first straight line L1 and the fifth straight line L5. As such, in a case in which the first angle θ1 formed between the first straight line L1 and the second straight line L2 is less than the fourth angle θ4 formed between the first straight line L1 and the fifth straight line L5, the second ultrasound transducers are set to the fifth ultrasound transducers 41 and 79. The sound speed calculation device 18 calculates the sound speed in the region of interest 35 of the subject, using the ultrasound echo signals output from the first ultrasound transducer 60, the set second ultrasound transducers 41 and 79, other ultrasound transducers 42 to 59 between the second ultrasound transducer 41 and the first ultrasound transducer 60, and other ultrasound transducers 61 to 79 between the first ultrasound transducer 60 and the second ultrasound transducer 79.

In Fig. 17, the control device 2 (sound speed calculation means) does not necessarily clearly set the second ultrasound transducers to the fifth ultrasound transducers. In a case in which the first angle θ1 is less than the third angle θ3 and the first angle θ1 is less than the fourth angle θ4, the sound speed in the region of interest 35 of the subject may be calculated using the ultrasound echo signals output from the ultrasound transducers 41 to 79 in the range interposed between two fourth straight lines L4 (the range included in the fourth angle θ4 formed between the first straight line L1 and the fourth straight line L4).

In a case in which the first angle θ1 is equal to the fourth angle θ4, the sound speed in the region of interest 35 of the subject may be calculated as illustrated in Fig. 16 or the sound speed in the region of interest 35 of the subject may be calculated as illustrated in Fig. 17.

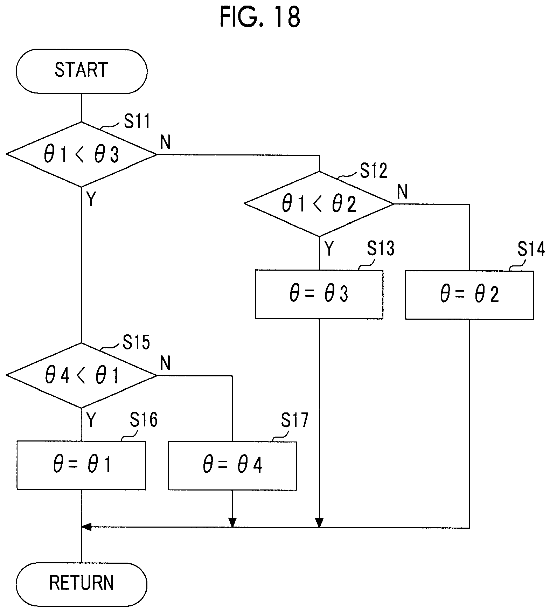

Fig. 18 is a flowchart illustrating the procedure of a process of determining an angle θ used according to the first to fourth angles θ1 to θ4. The sound speed in the region of interest of the subject is calculated using the ultrasound echo signals output from the ultrasound transducers included in the range of the determined angle θ used.

In a case in which the first angle θ1 is equal to or greater than the third angle θ3 (NO in Step S11), the control device 2 determines whether the first angle θ1 is less than the second angle θ2 (Step S12). In a case in which the first angle θ1 is less than the second angle θ2 (YES in Step S12), the angle θ used is determined to be the third angle θ3 (Step S13). In a case in which the first angle θ1 is equal to or greater than the second angle θ2 (NO in Step S12), the angle θ used is determined to be the second angle θ2 (Step S14).

In a case in which the first angle θ1 is less than the third angle θ3 (YES in Step S11), the control device 2 determines whether the fourth angle θ4 is less than the first angle θ1 (Step S15). In a case in which the fourth angle θ4 is less than the first angle θ1 (YES in Step S15), the angle θ used is determined to be the first angle θ1 (Step S16). In a case in which the fourth angle θ4 is equal to or greater than the first angle θ1 (NO in Step S15), the angle θ used is determined to be the fourth angle θ4 (Step S17).

In a case in which the first angle θ1 is equal to the third angle θ3 in Step S11 of Fig. 18, the process may proceed to Step S5. In a case in which the first angle θ1 is equal to the second angle θ2 in Step S12, the angle θ used may be determined to be the third angle θ3. In a case in which the fourth angle θ4 is equal to the first angle θ1 in Step S15, the angle θ used may be determined to be the first angle θ1.

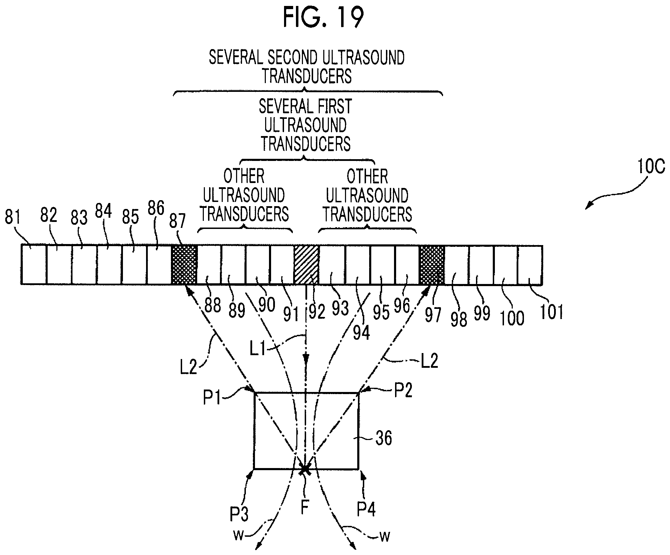

Fig. 19 illustrates another embodiment and illustrates an aspect in which an ultrasound probe 10C transmits the ultrasonic waves w to the subject.

The ultrasound probe 10C is a linear ultrasound probe 10C. The ultrasound probe 10C includes a plurality of ultrasound transducers 81 to 101 which are arranged in a line.

The transmission direction of the ultrasound probe 10C is perpendicular to the direction in which the ultrasound transducers 81 to 101 of the ultrasound probe 10C are arranged and is oriented to the subject. A region of interest 36 of the subject is set. The region of interest 36 of the subject has a rectangular shape. The focus F is set in a portion that is farthest in the transmission direction in the region of interest 36 of the subject.

The several first ultrasound transducers 90 to 94 transmit the ultrasonic waves w to the subject and the several second ultrasound transducers 87 to 97 receive ultrasound echoes from the focus F. The first ultrasound transducers 90 to 94 may be the same as the second ultrasound transducers 87 to 97. All of the plurality of ultrasound transducers 81 to 101 included in the ultrasound probe 10C may be the several first ultrasound transducers or the several second ultrasound transducers. All of the plurality of ultrasound transducers 81 to 101 included in the ultrasound probe 10C may be the several first ultrasound transducers and the several second ultrasound transducers.

A straight line connecting the focus F and a first ultrasound transducer 92 that is closest to the focus F is the first straight line L1. In addition, straight lines connecting the focus F and two first vertexes P1 and P2 which are farthest from the focus F among four vertexes P1, P2, P3, and P4 of the region of interest 36 of the subject are the second straight lines L2.

The sound speed calculation device 18 calculates the sound speed in the region of interest 36 of the subject, using the ultrasound echo signals output from the first ultrasound transducer 60 that is closest to the focus F among the several second ultrasound transducers 87 to 97 receiving the ultrasound echoes from the set focus F, two second ultrasound transducers 87 and 97 which are the second ultrasound transducers other than the first ultrasound transducer 60, are located on the straight lines L2 passing through the focus F and the region of interest 35 of the subject, and have the first ultrasound transducer 60 interposed therebetween, other ultrasound transducers 88 to 91 between the first ultrasound transducer 50 and the second ultrasound transducer 87, the first ultrasound transducer 50, and the second ultrasound transducer 96.

### Explanation of References

1: ultrasound diagnostic apparatus (sound speed calculation system)
2: control device (transmission control means, sound speed calculation means, focus setting means, sound speed calculation means)
3: operation device (region-of-interest setting means)
5: operation control device (transmission control means)
7: transmission control device (transmission control means)
8: driving signal generation device (transmission control means)
10, 10A, 10B, 10C: ultrasound probe (acoustic probe)
15: STC device (acoustic image generation means)
16: DSC (acoustic image generation means)
18: sound speed calculation device (sound speed calculation means)

## Claims

1. A sound speed calculation system comprising:
an acoustic probe in which a plurality of acoustic transducers are arranged;
transmission driving means for driving the acoustic transducers such that the acoustic transducers transmit acoustic waves to a subject;
acoustic image generation means for generating an acoustic image of the subject, using acoustic echo signals output from the acoustic transducers that transmit the acoustic waves to the subject and receive acoustic echoes from the subject;
region-of-interest setting means for setting a region of interest in the acoustic image generated by the acoustic image generation means;
focus setting means for setting a focus of the acoustic waves transmitted from several first acoustic transducers among the plurality of acoustic transducers in a portion that is farthest in a transmission direction of the acoustic waves in a region of interest of the subject which corresponds to the region of interest of the acoustic image; and
sound speed calculation means for calculating a sound speed in the region of interest of the subject, using the acoustic echo signals output from a first acoustic transducer which is closest to the focus among several second acoustic transducers receiving the acoustic echoes from the focus set by the focus setting means, two second acoustic transducers which are other than the first acoustic transducer, are located on straight lines passing through the focus and the region of interest of the subject, and have the first acoustic transducer interposed therebetween, and other acoustic transducers between the first acoustic transducer and the second acoustic transducers.

2. The sound speed calculation system according to claim 1,
wherein the region of interest of the subject has a shape that is surrounded by two radii and arcs of a circle, and
the portion that is farthest in the transmission direction of the acoustic waves is a center of the arc which is farthest from the acoustic probe in the region of interest of the subject.

3. The sound speed calculation system according to claim 2,
wherein, in a case in which a straight line connecting the focus and the first acoustic transducer is a first straight line and a straight line connecting the focus and a first vertex that is farthest from the focus among four vertexes of the region of interest of the subject is a second straight line, the sound speed calculation means sets the second acoustic transducer to an acoustic transducer located on the second straight line and calculates the sound speed in the region of interest of the subject, using the acoustic echo signals output from the first acoustic transducer, the second acoustic transducer, and other acoustic transducers.

4. The sound speed calculation system according to claim 3,
wherein, in a case in which a straight line connecting the focus and a third acoustic transducer that is farthest from the focus among the plurality of acoustic transducers is a third straight line, the sound speed calculation means sets the second acoustic transducer to the acoustic transducer located on the second straight line and calculates the sound speed in the region of interest of the subject, using the acoustic echo signals output from the first acoustic transducer, the second acoustic transducer, and other acoustic transducers, in a case in which a first angle formed between the first straight line and the second straight line is less than a second angle formed between the first straight line and the third straight line, and
the sound speed calculation means sets the second acoustic transducer to the third acoustic transducer and calculates the sound speed in the region of interest of the subject, using the acoustic echo signals output from the first acoustic transducer, the second acoustic transducer, and other acoustic transducers, in a case in which the first angle is greater than the second angle.

5. The sound speed calculation system according to claim 3,
wherein the several second acoustic transducers are a maximum number of acoustic transducers that are capable of receiving the acoustic echoes used to calculate the sound speed in the region of interest of the subject from the set focus at the same time,
in a case in which a straight line connecting the focus and a fourth acoustic transducer that is farthest from the focus among the several second acoustic transducers is a fourth straight line, the sound speed calculation means sets the second acoustic transducer to the acoustic transducer located on the second straight line and calculates the sound speed in the region of interest of the subject, using the acoustic echo signals output from the first acoustic transducer, the second acoustic transducer, and other acoustic transducers, in a case in which a first angle formed between the first straight line and the second straight line is less than a third angle formed between the first straight line and the fourth straight line, and
the sound speed calculation means sets the second acoustic transducer to the fourth acoustic transducer and calculates the sound speed in the region of interest of the subject, using the acoustic echo signals output from the first acoustic transducer, the second acoustic transducer, and other acoustic transducers, in a case in which the first angle is greater than the third angle.

6. The sound speed calculation system according to claim 3,
wherein the several second acoustic transducers include a fifth acoustic transducer,
the fifth acoustic transducer is configured such that a difference between a first distance between the focus and the first acoustic transducer and a second distance between the focus and the fifth acoustic transducer is closest to a predetermined detection limit width,
in a case in which a straight line connecting the focus and a fourth acoustic transducer that is farthest from the focus among the several second acoustic transducers is a fifth straight line, the sound speed calculation means sets the second acoustic transducer to the acoustic transducer located on the second straight line and calculates the sound speed in the region of interest of the subject, using the acoustic echo signals output from the first acoustic transducer, the second acoustic transducer, and other acoustic transducers, in a case in which a first angle formed between the first straight line and the second straight line is greater than a fourth angle formed between the first straight line and the fifth straight line, and
the sound speed calculation means sets the second acoustic transducer to the fifth acoustic transducer and calculates the sound speed in the region of interest of the subject, using the acoustic echo signals output from the first acoustic transducer, the second acoustic transducer, and other acoustic transducers, in a case in which the first angle is less than the fourth angle.

7. The sound speed calculation system according to claim 3,
wherein the several second acoustic transducers are a maximum number of acoustic transducers that are capable of receiving the acoustic echoes used to calculate the sound speed in the region of interest of the subject from the set focus at the same time,
in a case in which a straight line connecting the focus and a third acoustic transducer that is farthest from the focus among the plurality of acoustic transducers is a third straight line, a straight line connecting the focus and a fourth acoustic transducer that is farthest from the focus among the several second acoustic transducers is a fourth straight line, and a first angle formed between the first straight line and the second straight line is greater than a third angle formed between the first straight line and the fourth straight line, the sound speed calculation means sets the second acoustic transducer to the fourth acoustic transducer and calculates the sound speed in the region of interest of the subject, using the acoustic echo signals output from the first acoustic transducer, the second acoustic transducer, and other acoustic transducers, in a case in which the first angle formed between the first straight line and the second straight line is less than a second angle formed between the first straight line and the third straight line, and
the sound speed calculation means sets the second acoustic transducer to the third acoustic transducer and calculates the sound speed in the region of interest of the subject, using the acoustic echo signals output from the first acoustic transducer, the second acoustic transducer, and other acoustic transducers, in a case in which the first angle is greater than the second angle.

8. The sound speed calculation system according to claim 3,
wherein the several second acoustic transducers are a maximum number of acoustic transducers that are capable of receiving the acoustic echoes used to calculate the sound speed in the region of interest of the subject from the set focus at the same time,
the several second acoustic transducers include a fifth acoustic transducer,
the fifth acoustic transducer is configured such that a difference between a first distance between the focus and the first acoustic transducer and a second distance between the focus and the fifth acoustic transducer is closest to a predetermined detection limit width,
in a case in which a straight line connecting the focus and a third acoustic transducer that is farthest from the focus among the plurality of acoustic transducers is a third straight line, a straight line connecting the focus and a fourth acoustic transducer that is farthest from the focus among the several second acoustic transducers is a fourth straight line, a straight line connecting the focus and the fourth acoustic transducer that is farthest from the focus among the several second acoustic transducers is a fifth straight line, and a first angle formed between the first straight line and the second straight line is less than a third angle formed between the first straight line and the fourth straight line, the sound speed calculation means sets the second acoustic transducer to the acoustic transducer located on the second straight line and calculates the sound speed in the region of interest of the subject, using the acoustic echo signals output from the first acoustic transducer, the second acoustic transducer, and other acoustic transducers, in a case in which the first angle formed between the first straight line and the second straight line is greater than a fourth angle formed between the first straight line and the fifth straight line, and
the sound speed calculation means sets the second acoustic transducer to the fifth acoustic transducer and calculates the sound speed in the region of interest of the subject, using the acoustic echo signals output from the first acoustic transducer, the second acoustic transducer, and other acoustic transducers, in a case in which the first angle is less than the fourth angle.

9. The sound speed calculation system according to claim 2,
wherein the acoustic probe is a convex acoustic probe in which the plurality of acoustic transducers are arranged in an arc shape.

10. The sound speed calculation system according to claim 1,
wherein the acoustic probe is a linear acoustic probe,
the region of interest of the subject has a rectangular shape, and
the portion that is farthest in the transmission direction of the acoustic waves is a center of a side that is far away from the acoustic probe in the transmission direction of the acoustic waves in the region of interest of the subject.

11. A sound speed calculation method comprising:
allowing transmission driving means to drive a plurality of acoustic transducers included in an acoustic probe in which the acoustic transducers are arranged such that the acoustic transducers transmit acoustic waves to a subject;
allowing acoustic image generation means to generate an acoustic image of the subject, using acoustic echo signals output from the acoustic transducers that transmit the acoustic waves to the subject and receive acoustic echoes from the subject;
allowing region-of-interest setting means to set a region of interest in the acoustic image generated by the acoustic image generation means;
allowing focus setting means to set a focus of the acoustic waves transmitted from several first acoustic transducers among the plurality of acoustic transducers in a portion that is farthest in a transmission direction of the acoustic waves in a region of interest of the subject which corresponds to the region of interest of the acoustic image; and
allowing sound speed calculation means to calculate a sound speed in the region of interest of the subject, using the acoustic echo signals output from a first acoustic transducer which is closest to the focus among several second acoustic transducers receiving the acoustic echoes from the focus set by the focus setting means, two second acoustic transducers which are other than the first acoustic transducer, are located on straight lines passing through the focus and the region of interest of the subject, and have the first acoustic transducer interposed therebetween, and other acoustic transducers between the first acoustic transducer and the second acoustic transducers.
